# EUROPEAN PATENT APPLICATION

(11) **EP 4 302 751 A1**
(43) Date of publication of application: **10.01.2024**
(21) Application number: 22183691.9
(22) Date of filing: 07.07.2022
(51) Int. Cl.: A61K 9/00, A61K 9/70, A61K 31/137, A61P 1/00, A61P 37/08, A61K 47/32, A61K 47/34, A61K 47/38, A61K 47/10, A61K 47/20, A61K 31/417

(54) **ORAL THIN FILMS WITH LOW WATER ACTIVITY**

(71) Applicant: LTS Lohmann Therapie-Systeme AG, 56626 Andernach (DE); ALK-Abelló A/S, 2970 Hørsholm (DK)
(72) Inventor: FICKER, Mario, 53129 Bonn (DE); LINN, Michael, 55596 Waldböckelheim (DE); MÜLLER, Markus, 53840 Troisdorf (DE); NORELLI, Claudia, 56204 Hillscheid (DE); WARNUS, Sabine, 56729 Ettringen (DE); RICHTER-FRIIS, Martin, 2970 Hørsholm (DK)
(74) Representative: Meissner Bolte Partnerschaft mbB

(57) **Abstract**

Oral thin film comprising adrenaline or a pharmaceutically acceptable salt thereof and at least one polymer, characterized in that the water activity of the oral thin film is less than 0.6.

Method for preparing said oral thin film, comprising the steps of

a) preparing a solution, dispersion or melt comprising adrenaline or a pharmaceutically acceptable salt thereof and at least one polymer and

b) spreading the solution, dispersion or melt from step a) or the optionally foamed solution, dispersion or melt from step al) in order to obtain an oral thin film, and

(c) drying the oral thin film obtained in step (b) in order to obtain an oral thin film having a water activity of less than 0.6.

Said oral thin film for use as a medicament, particularly for use as a medicament in the treatment of allergic reactions.

## Description

The present invention is directed to an oral thin film, a process for its preparation and its use as a medicament.

Oral thin films (OTF) are thin films containing at least one pharmaceutically active ingredient that are placed directly in the oral cavity or applied to the oral mucosa and dissolve there. In particular, these are thin polymer-based films containing at least one active ingredient which, when applied to a mucosa, especially the oral mucosa, release the active ingredient directly into it. The very good blood circulation of the oral mucosa ensures a rapid transfer of the active ingredient into the blood circulation. This dosage system has the advantage that the active ingredient is largely absorbed through the mucosa, thus avoiding the "first pass metabolism" that occurs with the conventional dosage form of an active ingredient in tablet form.

A problem with the administration of adrenaline, especially in dissolved form, is that only the L-enantiomer of adrenaline is pharmaceutically active. However, due to the influence of oxygen and water, pure L-adrenaline racemizes rapidly, whereby the pharmaceutical activity of known dosage forms rapidly decreases. Therefore, it is tried to counteract this racemization by adding antioxidants. As an antioxidant sodium metabisulfite is usually used, often in a ratio of 1:1 to adrenaline. However, this has the disadvantage that the adrenaline reacts with the sodium metabisulfite to form undesirable adrenaline sulfonate.

While the degradation of adrenalin to adrenochrome leads to a strong coloration, the degradation mechanisms to D-adrenalin and adrenaline sulfonate are responsible for the quantitatively largest degradation. This occurs via a planar sp²-transition state which is stabilized by the activated aromatic. Reactions of the transition state with water lead to the formation of D- and L-adrenaline in a ratio of 1:1, which in the long term leads to a racemization of the starting material. The large use of sodium metabisulfite in the existing market products (Adrenaline^{®} ratio 1:1, Epipene ratio 1.7:1, related to sodium metabisulfite to adrenaline) leads to the reaction of sulfite (SO₃²⁻) and disulfite (S₂O₅²⁻) with adreanline (S_{N}¹ reaction). At the end of shelf-life, known market products therefore contain up to 15% adrenaline sulfonate.

US 2018/0125977 A1 discloses pharmaceutical compositions for the administration of adrenaline, wherein the adrenaline is partially dissolved in the composition.

The problem underlying the present invention is to overcome the aforementioned disadvantages of the prior art. In particular, the problem of the present invention is to provide a dosage form for adrenaline in which the degradation and/or racemization of the pharmaceutically active L-adrenalin is prevented to the greatest possible extent without the need to use large amounts of antioxidants, in particular sodium metabisulfite. Furthermore, the dosage form should also be as stable as possible with regard to the possible growth of microorganisms without the need to use large quantities of antimicrobial additives. Moreover, the dosage form should be easy and as inexpensive as possible to produce.

This problem is solved by an oral thin film according to claim 1, which comprises adrenaline or a pharmaceutically acceptable salt thereof and at least one polymer, wherein the water activity of the oral thin film is less than 0.6.

Such a dosage form overcomes the above-mentioned disadvantages. Due to the polymer matrix of the oral thin film, the active ingredient adrenaline is protected from oxygen and extrinsic moisture. At the same time, the extremely low water activity largely prevents the racemization of L-adrenaline, even without using large amounts of antioxidants, especially sodium metabisulfite. Since no more growth of microorganisms takes place at water activities of less than 0.6 essentially no antimicrobial additives are needed in the oral thin film.

Water activity is a parameter to compare aqueous solutions and solids (e.g. pharmaceuticals or food) regarding the availability of water for chemical, biochemical and microbial reactions.

The water activity is expressed as the a_{w} value and is defined as the quotient of the concentration of water in the vapor phase in the airspace above the material and the concentration of water in the airspace above pure water at a given temperature. The water activity corresponds to 1/100 of the relative equilibrium humidity (RGF).

The relationship between water content and water activity of a substrate is represented by its sorption isotherm. Decreasing water activity first slows down the growth of microorganisms, followed by enzymatic reactions and finally non-enzymatic reactions.

Since the water requirements of the various organisms are well known, the a_{w} value can be used to determine the shelf life. It has been established that substrates with an a_{w} value below 0.60 are considered to have an unlimited shelf life.

The oral thin film according to the invention has a water activity of less than 0.6, preferably less than 0.55, preferably less than 0.50, preferably less than 0.45, preferably less than 0.40 and more preferably less than 0.35.

Water activity of the oral thin film is thereby determined using a Novasina LabMaster-aw neo G00572. The device is switched on at least one day before the measurement for reasons of equilibration. Three samples are put together in a Novasina sample cup (P/N 2601518) and analyzed immediately afterwards.

Preferably, the oral thin film according to the invention can also contain no water at all and thus have a water activity of zero.

Adrenaline or epinephrine is a hormone produced in the adrenal medulla which belongs to the group of catecholamines. Adrenaline is also found in the central nervous system, where it is present as a neurotransmitter in adrenergic nerve cells. Adrenaline mediates its effects by activating G protein-coupled receptors, the adrenoceptors.

Adrenaline is also known under the systematic IUPAC name 4-[1-hydroxy-2-(methylamino)ethyl]benzene-1,2-diol. Only the L-enantiomer is pharmaceutically active.

The oral thin film according to the invention is therefore preferably characterized in that the adrenalin or the pharmaceutically acceptable salt thereof is present as L-enantiomer.

The adrenaline is preferably present as adrenaline hydrogentartrate, in particular as L-adrenaline hydrogentartrate.

The oral thin film according to the invention is preferably characterized in that the at least one polymer comprises at least one water-soluble polymer.

Water-soluble polymers comprise chemically different, natural or synthetic polymers whose common characteristic is their solubility in water or aqueous media. A prerequisite is that these polymers have a sufficient number of hydrophilic groups for water solubility and are not cross-linked. The hydrophilic groups can be non-ionic, anionic, cationic and/or zwitterionic.

Preferably the at least one polymer in the oral thin film of the invention is selected from the group consisting of starch and starch derivatives, dextrans, cellulose derivatives such as carboxymethyl cellulose, hydroxypropyl cellulose, hydroxyethyl cellulose, hydroxypropyl methyl cellulose, hydroxypropylethyl cellulose, sodium carboxymethyl cellulose, ethyl or propyl cellulose, polyacrylic acids, polyacrylates, polyvinyl pyrrolidones, polyvinyl alcohols, polyvinyl pyrrolidone/polyvinyl acetate copolymers, polyethylene oxide polymers, polyacrylamides, polyethylene glycols, polyvinyl alcohol/polyethylene glycol co-polymers, gelatine, collagen, alginates, pectin, pullulan, tragacanth, chitosan, alginic acid, arabinogalactan, galactomannan, agar, agarose, carrageenan, natural gums and mixtures thereof.

Particularly preferred are hydroxypropyl cellulose, polyvinyl pyrrolidones, polyvinyl pyrrolidone/polyvinyl acetate copolymers, polyvinyl alcohol, polyvinyl alcohol/polyethylene glycol co-polymers and/or pectin (amylopectin).

The oral thin film according to the invention is preferably characterized in that the oral thin film contains less than 1% by weight, preferably less than 0.5% by weight, more preferably less than 0.1% by weight, based on the total weight of the oral thin film, of antioxidants.

Known antioxidants or stabilizers that are normally used include in particular tocopherols and their esters, sesamol from sesame oil, coniferous benzoate from benzoin resin, nordihydroguaiethic resin and nordihydroguaiaretic acid (NDGA), gallates (inter alia methyl, ethyl, propyl, amyl, butyl, lauryl gallates), butylated hydroxyanisole (BHA/BHT, also butyl-p-cresol), ascorbic acid and salts and esters thereof (e.g. acorbyl palmitate), erythorbic acid (isoascorbinic acid) and salts and esters thereof, monothioglycerol, sodium formaldehyde sulfoxylate, sodium metabisulfite, sodium bisulfite, sodium sulfite, potassium metabisulfite, butyl hydroxyanisole, butyl hydroxytoluene (BHT), and/or propionic acid.

The largely absence of antioxidants has the advantage that the antioxidants cannot react with the active ingredient and produce undesirable compounds. Furthermore, antioxidants can trigger allergic reactions. The absence or a reduction of antioxidants is therefore advantageous.

In particular, it is advantageous that the oral thin film of the invention contains less than 1% by weight, preferably less than 0.5% by weight, more preferably less than 0.1% by weight, of a metabisulfite, such as sodium metabisulfite or potassium metabisulfite, based on the total weight of the oral thin film.

Especially a low amount or even the absence of sodium metabisulfite is advantageous, as sodium metabisulfite can cause allergic reactions and can react with adrenaline to adrenaline sulfonate.

The oral thin film according to the invention is preferably characterized in that the oral thin film contains less than 5% by weight, preferably less than 4% by weight, preferably less than 3% by weight, preferably less than 2% by weight, preferably less than 1% by weight, preferably less than 0.5% by weight and more preferably less than 0.1% by weight of antimicrobial substances, based on the total weight of the oral thin film.

The absence of antimicrobial substances has the advantage that antimicrobial substances may also be toxic to the patient or may cause allergic reactions. This risk is reduced by dispensing with antimicrobial substances as far as possible.

Known antimicrobial substances comprise especially phyto-extracts.

The oral thin film according to the invention is therefore preferably characterized in that the oral thin film contains less than 5% by weight, preferably less than 4% by weight, preferably less than 3% by weight, preferably less than 2% by weight, preferably less than 1% by weight, preferably less than 0.5% by weight and more preferably less than 0.1% by weight of phyto-extract, based on the total weight of the oral thin film.

The oral thin film according to the invention is preferably characterized in that the oral thin film contains less than 10% by weight, preferably less than 9% by weight, preferably less than 8% by weight, preferably less than 7% by weight, preferably less than 6% by weight, preferably less than 5% by weight and more preferably less than 3% by weight of water, based on the total weight of the oral thin film.

In order to determine the water content, the oral thin film is analyzed via coulometric Karl Fischer titration after dissolution in 5 mL of dimethyl sulfoxide through shaking on a laboratory shaker at about 1200 rpm for 10 min.

The oral thin film according to the invention may however comprise up to 10% by wt., preferably up to 4% by wt. of an organic solvent, such as ethanol and/or acetone.

The oral thin film of the invention is preferably characterized in that the oral thin film contains at least 30% by weight, preferably at least 45% by weight and more preferably at least 50% by weight of adrenaline or a pharmaceutically acceptable salt thereof (as defined above), based on the total weight of the oral thin film.

Preferably, the oral thin film according to the invention is characterized in that the oral thin film contains 40% by weight to 65% by weight, preferably 45% to 60% by weight and more preferably 50% by weight to 56% by weight of adrenaline or a pharmaceutically acceptable salt thereof (as defined above), based on the total weight of the oral thin film.

The oral thin film of the invention is preferably characterized in that the oral thin film contains at least 30% by weight to 60by weight, preferably 33% by weight to 55 % by weight, preferably 35% by weight to 50% by weight, preferably 38% by weight to44% by weight of the at least one polymer, based on the total weight of the oral thin film.

Blends of several polymers can also be used. In this case the sum of all polymers used is preferably at least 30% by weight to 60 % by weight, preferably at least 35% by weight to 55 % by weight, preferably at least 38% by weight to 51% by weight, preferably at least 39% by weight to 50% by weight, based on the total weight of the oral thin film.

The oral thin film according to the invention is preferably characterized in that the oral thin film further comprises at least one excipient selected from the group consisting of dyes, flavors, sweeteners, taste masking agents, emulsifiers, enhancers, pH regulators, humectants and/or preservatives.

The excipients may each be present in the oral thin film in an amount of about 0.001 to 10% by weight, preferably 0.01 to 5% by weight, preferably 0.01 to 2% by weight, based on the total weight of the oral thin film.

In one embodiment, the oral thin film according to the invention is present in the form of an essentially smooth film.

In another embodiment, the oral thin film is present in the form of a solidified foam comprising cavities.

The cavities and the associated larger surface area of the films facilitate in particular the entry of water, saliva or other body fluids into the interior of the film and thus accelerate the dissolution of the dosage form and the release of the active ingredient.

In the case of a fast-absorbing drug, transmucosal absorption can also be improved by the rapid dissolution of the oral thin film.

Further, the wall thickness of the cavities mentioned is preferably small, as they represent for example solidified bubbles, so that a rapid dissolution or destruction of these cavities takes place.

A further advantage of this embodiment is that, despite the comparatively high weight per unit area, faster drying can be achieved by converting the oral thin film into a foam compared to non-foamed oral thin films.

Preferably, the oral thin film according to the invention is characterized in that the cavities are isolated from each other and are preferably present in the form of bubbles, wherein the cavities are preferably filled with air or a gas, preferably an inert gas, particularly preferably nitrogen, carbon dioxide, helium or a mixture of at least two of these gases.

In another embodiment, the cavities are able to communicate with each other, preferably by forming a continuous channel system penetrating the oral thin film.

Preferably, the cavities mentioned have a volume percentage of 5 to 98 %, preferably of 50 to 80 %, based on the total volume of the oral thin film. In this way, the advantageous effect of accelerating the dissolution of the oral thin film is favorably influenced.

Surface active agents and surfactants, respectively may be added to the oral thin film in order to form the foam or can be added to the foam obtained before or after drying to improve the stability of the foam before or after drying.

Another parameter that influences the properties of the oral thin film according to the invention is the diameter of the cavities or bubbles. The bubbles or cavities are preferably produced with the aid of a foam-whipping machine with which the diameter of the bubbles can be adjusted over a wide range, almost at will. Thus, the diameter of the bubbles or cavities can range from 0.01 to 60 µm. Especially preferred is a diameter in the range of 10 and 50 µm.

The oral thin film according to the invention is preferably characterized in that the oral thin film has an area weight of 20 to 400 g/m², preferably of 100 to 350 g/m².

The oral thin film according to the invention is further preferably characterized in that the oral thin film comprises at least one material, which serves as a crystallization seed for adrenaline.

The oral thin film according to the invention is preferably characterized in that the material serving as the crystallization seed for adrenaline comprises small particles insoluble in the environment of the final oral thin film and also in the mass during the manufacture of the oral thin film.

In this context, small is understood to mean an average particle size of 5 to 100 µm, preferably of 20 to 50 µm, whereby the average particle size can be taken from the product data sheets of the respective manufacturers valid at the date of application.

The oral thin film according to the invention is particularly preferred characterized in that the material serving as the seed for adrenaline crystallization comprises amorphous fumed silica.

Amorphous fumed silica (SiO₂) is a synthetically produced colloidal silica. It consists essentially entirely of amorphous silica particles aggregated into larger units.

Suitable amorphous fumed silicas are known, for example, from the series with the trade name "Aerosil^{®}" from Evonik.

Also suitable as materials that can serve as crystallisation seeds are TiO₂, iron oxide(s), silicates, aluminium trioxide and/or magnesium stearate.

The oral thin film according to the invention is further preferably characterized in that after six months storage at 40°C less than 3% of the contained adrenaline has been degraded in the oral thin film and/or that less than 2.5% of the contained adrenaline has been racemized.

The present invention also concerns a method for producing the oral thin film according to the invention. The method comprising the steps:
a) preparing a solution, dispersion or melt comprising adrenaline or a pharmaceutically acceptable salt thereof and at least one polymer and
a1) optionally foaming the solution, dispersion or melt from step a) by introducing a gas or gas mixture, by chemical gas generation or by expansion of a dissolved gas,
b) spreading the solution, dispersion or melt from step a) or the optionally foamed solution, dispersion or melt from step a1) in order to obtain an oral thin film, and
(c) drying the oral thin film obtained in step (b) in order to obtain an oral thin film having a water activity of less than 0.6.

It is clear to the person skilled in the art that step a1) is only necessary if the oral thin film is to be in the form of a solidified foam comprising cavities.

All preferred embodiments described above for the oral thin film apply analogously to the method according to the invention.

Water or organic solvents such as ethanol, acetone and mixtures thereof are suitable solvents to be used in the method of the invention.

The water activity of less than 0.6 can be achieved in particular by extensive drying the formulations, especially the foam formulations. Due to the porous structure, the OTF can be dried well and achieves a low water proportion/water activity.

Furthermore, organic solvents are preferred as process solvents. In addition, the low water activity can be maintained by storage in suitable packaging material. In addition, the choice of less hygroscopic auxiliary materials and/or polymers is preferred.

Drying conditions for solvent evaporation can be e.g. in the range of 40 to 100 °C, more preferably 50 to 80 °C, most preferably 50 to 75°C. It is also possible that drying is effected by applying a temperature gradient.

The oral thin films are preferably dried for at least 10 min, preferably from 13 to 25 min.

The present invention is also directed to an oral thin film obtainable by the aforementioned method.

All preferred embodiments described above for the oral thin film and the method for obtaining the oral thin film apply analogously to the oral thin film obtainable by the method according to the invention.

The present invention is further directed at an oral thin film, as described above or obtainable by the above-mentioned method, for use as a medicament, in particular in the treatment of allergic reactions, in the treatment of shock conditions, in the use in emergency medicine, e.g. for resuscitation, treatment of respiratory diseases and/or for local vasoconstriction.

The invention is further explained by means of non-restrictive examples.

### Examples

Oral thin films with the compositions listed in Table 1 were prepared as follows:
Preparation of the foam formulations: 197Adr0086, 197Adr0078, 197Adr0091 and 197Adr0084

The production of the oral thin films can be carried out by techniques of mass production known to the person skilled in the art, e.g. by agitating/mixing the contained components by means of agitator motor and suitable stirring tools.

Gas is then added to the resulting mass by stirring, e.g. using a foam whipping machine. The foamy mass may then be coated in a constant layer thickness on a coating carrier by suitable equipment (roller applicator, doctor blade, coating box, etc.).

All temperature-stable web-shaped materials from which the dry film can be removed can serve as coating carrier. This can be achieved by material selection of the coating carrier (different surface tensions between foamed mass and substrate) or by suitable dehesive coatings of the coating carrier with e.g. silicones or fluoropolymers.

The process solvent(s) contained, usually water or mixtures of water and organic, water-miscible solvents, is/are removed by drying. From the solid foam layer thus obtained, the oral thin films can be cut or punched to the appropriate size. Drying is either achieved by drying the laminate in a ventilated drying oven or by moving the coated laminate through a ventilated drying channel. In case of the drying channel, the drying temperature is defined by the laminate speed and the length of thy drying channel, thus the duration of the laminate within the drying channel. The drying channel can be operated with a gradient of different temperature zone. In process controls, such as determination of water content, for example through Karl Fischer Titration, can be done to evaluate success of the drying step.

### Preparation of the suspension formulation 197Adr0100

The production can be carried out by techniques of mass production known to the person skilled in the art, e.g. by agitating/mixing the contained components by means of agitator motor and suitable stirring tools.

The mass obtained in this way is coated in a constant layer thickness onto a coating carrier by suitable equipment (roller applicator, doctor blade, coating box, etc.).

All temperature-stable web-shaped materials from which the dry film can be removed can serve as coating carrier. This can be achieved by material selection of the coating carrier (different surface tensions between mass and substrate) or by suitable dehesive coatings of the coating carrier with e.g. silicones or fluoropolymers.

The process solvent(s) contained in the coating is/are removed by drying.

Drying is either achieved by drying the laminate in a ventilated drying oven or by moving the coated laminate through a ventilated drying channel. In case of the drying channel, the drying temperature is defined by the laminate speed and the length of thy drying channel, thus the duration of the laminate within the drying channel. The drying channel can be operated with a gradient of different temperature zone. In process controls, such as determination of water content, for example through Karl Fischer Titration, can be done to evaluate success of the drying step.

From the solid laminate layer thus obtained, oral thin films can be cut or punched to the appropriate size. Alternatively, several laminates can be combined into a thicker laminate by laminating. This can be achieved by heating, high pressure or the use of adhesive laminates.

**Table 1:**

| **Material** | **197Adr0086** | **197Adr0078** | **197Adr0091** | **197Adr0084** | **197Adr0100** |
|---|---|---|---|---|---|
| Name | Kollicoat | PVA | Kollicoat + Phentolamin | Kollicoat + Proloc | Suspension |
| Adrenaline hydrogentartrate | 55.00% | 55.00% | 55.00% | 55.00% | 50.00% |
| Kollicoat IR¹ | 43.90% | - | 38.80% | 38.90% | - |
| PVA 4-88² | - | 39.90% | - | - | - |
| Kollidone VA64³ | - | - | - | - | 39.90% |
| HPC⁴ | - | - | - | - | 10.00% |
| Proloc 15⁵ | - | - | - | 5.00% | - |
| Phentolamine | - | - | 5.10% | - | - |
| Glycerol | 1.00% | 5.00% | 1.00% | 1.00% | - |
| Triacetine | - | - | - | - | - |
| Na-metabisulfit | 0.10% | 0.10% | 0.10% | 0.10% | 0.10% |
| Solvent | Aqua purificata | Aqua purificata | Aqua purificata | Aqua purificata | Ethanol |
| Area weight (dry) | 294 g/m² | 294 g/m² | 294 g/m² | 294 g/m² | 323.4 g/m² |
| | | | | | |
| Adrenaline hydrogentartrate mg / OTF size | 72.8 mg / 4.5 cm² | 72.8 mg / 4.5 cm² | 72.8 mg / 4.5 cm² | 72.8 mg / 4.5 cm² | 72.8 mg / 4.5 cm² |

| | | | | | |
|---|---|---|---|---|---|
| ^{1:} Polyvinyl alcohol/polyethylene glycol copolymer (from BASF) ^{2:} Polyvinyl alcohol 4-88 (from Merck) ^{3:} Polyvinylpyrrolidone/polyvinyl acetate copolymer (from BASF) ^{4:} Hydroxypropyl cellulose (from Ashland) ^{5:} Carbomer / Amylopectin (from Henkel) | | | | | |

For the oral thin films produced, the water content and water activity were measured. The results are summarized in Table 2.

To determine the water content, each OTF was analyzed via coulometric Karl Fischer titration after dissolution in 5 mL of dimethyl sulfoxide through shaking on a laboratory shaker at about 1200 rpm for 10 min.

Water activity of OTFs was determined using a Novasina LabMaster-aw neo G00572. The device was switched on at least one day before the measurement for reasons of equilibration. Three samples were put together in a Novasina sample cup (P/N 2601518) and analyzed immediately afterwards.

**Table 2:**

| **Formulation** | **197Adr0086** | **197Adr0078** | **197Adr0091** | **197Adr0084** | **197Adr0100** |
|---|---|---|---|---|---|
| Water content | 8.3% | 6.8% | 7.4% | 9.0% | 2.8% (+3.5% EtOH) |
| Water activity | 0.436 | 0.503 | n.a | 0.502 | 0.305 |

Furthermore, the degradation of adrenaline and the racemization after storage at 25°C and 40°C, respectively were measured by HPLC and by comparing the relative peak sized of the peak arising from the L- and the D-enantiomer, respectively.

The results are summarized in Tables 3 and 4.

**Table 3:**

| | | 25°C | | | | | | | | | |
|---|---|---|---|---|---|---|---|---|---|---|---|
| | | T = 0 | | T = 6w | | T = 3M | | T = 5M | | T = M | |
| | | Ret.Time | Rel.Area | Ret.Time | Rel.Area | Ret.Time | Rel.Area | Ret.Time | Rel.Area | Ret.Time | Rel.Area |
| | | min | % | min | % | min | % | min | % | min | % |
| 197Adr0100 | L-adr | 23.9 | 99.35 | 16.6 | 99.26 | 17.8 | 99.28 | 17.5 | 99.26 | | |
| | D-adr | 26.7 | 0.65 | 18.3 | 0.68 | 19.5 | 0.71 | 19.3 | 0.68 | | |
| 197Adr0086 | L-adr | 23.8 | 99.28 | 16.6 | 98.63 | 17.7 | 98.58 | 17.5 | 98.75 | | |
| | D-adr | 26.7 | 0.73 | 18.3 | 0.81 | 19.5 | 0.91 | 19.3 | 0.89 | | |
| | | | | 29.6 | 0.43 | 33.8 | 0.35 | 32.8 | 0.25 | | |
| | | | | | | | | | | | |
| 197Adr0078 | | | | 12.6 | 0.22 | 12 | 0.15 | | | | |
| | L-adr | 23.9 | 98.85 | 16.6 | 97.35 | 17.8 | 98.61 | 17.5 | 98.60 | | |
| | D-adr | 26.7 | 1.15 | 18.3 | 1.34 | 19.5 | 1.03 | 19.4 | 1.07 | | |
| | | | | 29.6 | 0.92 | 33.9 | 0.24 | 33.0 | 0.21 | | |
| | | | | | | | | | | | |
| 197Adr0084 | L-adr | 23.9 | 99.24 | 16.6 | 98.75 | 17.8 | 98.66 | 17.6 | 98.69 | | |
| | D-adr | 26.8 | 0.77 | 18.3 | 0.82 | 19.5 | 0.95 | 19.3 | 0.96 | | |
| | | | | | | 33.9 | 0.32 | 33.0 | 0.24 | | |

**Table 4:**

| Batch no. | Formulation | | | 40°C | | | | | | | | | | | |
|---|---|---|---|---|---|---|---|---|---|---|---|---|---|---|---|
| | | | | T = 0 | | T = 2w | | T = 6w | | T = 3M | | T=5M | | T = 6M | |
| | | | | Ret.Time | Rel.Area | Ret.Time | Rel.Area | Ret.Time | Rel.Area | Ret.Time | Rel.Area | Ret.Time | Rel.Area | Ret.Time | Rel.Area |
| | | | | min | % | min | % | min | % | min | % | min | % | min | % |
| 197Adr0100 | Suspension | | | 23.9 | 99.35 | 24.3 | 99.38 | 16.6 | 99.26 | 17.8 | 99.20 | 13.3 | 0.06 | | |
| | | 71 | L-adr | | | | | | | | | 17.6 | 99.26 | | |
| | | | D-adr | 26.7 | 0.65 | 27.2 | 0.62 | 18.3 | 0.66 | 19.6 | 0.72 | 19.4 | 0.68 | | |
| 197Adr0086 | Kollicoat | 55 | | | | | | 12.6 | 0.11 | | | | | | |
| | | | L-adr | 23.8 | 99.28 | 24.3 | 99.02 | 16.6 | 98.41 | 17.8 | 98.33 | 17.6 | 97.96 | | |
| | | | D-adr | 26.7 | 0.73 | 27.2 | 0.86 | 18.3 | 1.06 | 19.6 | 1.3 | 19.4 | 1.56 | | |
| | | | | | | | | 29.7 | 0.28 | 34.2 | 0.18 | 33.4 | 0.21 | | |
| 197Adr0078 | PVA | 53 | | | | 10.6 | 0.12 | | | | | | | | |
| | | | | | | 18 | 0.15 | 12.9 | 0.13 | 12.1 | 0.15 | 11.8 | 0.20 | | |
| | | | L-adr | 23.9 | 98.85 | 24.2 | 98.30 | 16.6 | 97.73 | 17.9 | 97.62 | 17.7 | 96.96 | | |
| | | | D-adr | 26.7 | 1.15 | 27.2 | 1.40 | 18.3 | 1.58 | 19.6 | 1.89 | 19.4 | 2.33 | | |
| | | | | | | | | 29.7 | 0.31 | 32 | 0.12 | 31.7 | 0.11 | | |
| | | | | | | | | | | 34 | 0.16 | 33.4 | 0.20 | | |
| | | | | | | | | | | | | 11.8 | 0.11 | | |
| 197Adr0084 | Proloc + kollicoat | 54 | L-adr | 23.9 | 99.24 | 24.3 | 99.04 | 16.6 | 98.57 | 17.9 | 98.10 | 17.7 | 97.45 | | |
| | | | D-adr | 26.8 | 0.77 | 27.2 | 0.87 | 18.3 | 1.09 | 19.6 | 1.58 | 19.4 | 2.04 | | |
| | | | | | | | | 29.8 | 0.22 | 34.3 | 0.14 | 33.5 | 0.19 | | |

At 40°C after 5 months (values indicate change with respect to initial value)
197Adr0100: 0.09% reduction, of which 0.03% racemization
197Adr0086: 1.32% reduction, of which 0.83% is racemization
197Adr0078: 1.89% reduction, of which 1.18% racemization
197Adr0084: 1.79% reduction, of which 1.27% is racemization

## Claims

1. Oral thin film comprising adrenaline or a pharmaceutically acceptable salt thereof and at least one polymer, **characterized in that** the water activity of the oral thin film is less than 0.6.

2. Oral thin film according to claim 1, **characterized in that** the adrenaline or the pharmaceutically acceptable salt thereof is present as L-enantiomer.

3. Oral thin film according to any of the preceding claims, **characterized in that** the at least one polymer comprises at least one water-soluble polymer.

4. Oral thin film according to any of the preceding claims, **characterized in that** the at least one polymer is selected from the group consisting of starch and starch derivatives, dextrans, cellulose derivatives such as carboxymethyl cellulose, hydroxypropyl cellulose, hydroxyethyl cellulose, hydroxypropylmethyl cellulose, hydroxypropylethyl cellulose, sodium carboxymethyl cellulose, ethyl or propyl cellulose, polyacrylic acids, polyacrylates, polyvinylpyrrolidones, polyvinyl alcohols, polyvinylpyrrolidone/polyvinyl acetate copolymers, polyethylene oxide polymers, polyacrylamides, polyethylene glycols, polyvinyl alcohol/polyethylene glycol co-polymers, gelatine, collagen, alginates, pectin, pullulan, tragacanth, chitosan, alginic acid, arabinogalactan, galactomannan, agar, agarose, carrageenan, natural gums and mixtures thereof.

5. Oral thin film according to any of the preceding claims, **characterized in that** the oral thin film contains less than 1% by weight of antioxidants, in particular less than 1% by weight of a metabisulfite, based on the total weight of the oral thin film.

6. Oral thin film according to any of the preceding claims, **characterized in that** the oral thin film contains less than 5% by weight of antimicrobial substances, based on the total weight of the oral thin film.

7. Oral thin film according to any of the preceding claims, **characterized in that** the oral thin film contains less than 10% by weight of water, based on the total weight of the oral thin film.

8. Oral thin film according to any of the preceding claims, **characterized in that** the oral thin film contains at least 30% by weight of adrenaline or a pharmaceutically acceptable salt thereof, based on the total weight of the oral thin film.

9. Oral thin film according to any of the preceding claims, **characterized in that** the oral thin film contains at least 35% by weight of the at least one polymer, based on the total weight of the oral thin film.

10. Oral thin film according to any of the preceding claims, **characterized in that** the oral thin film further comprises at least one excipient selected from the group consisting of dyes, flavors, sweeteners, taste masking agents, emulsifiers, enhancers, pH regulators, humectants and/or preservatives.

11. Oral thin film according to any of the preceding claims, **characterized in that** the oral thin film has a surface weight of 20 to 400 g/m².

12. Oral thin film according to any of the preceding claims, **characterized in that** after six months storage at 40°C less than 3% of the contained adrenaline has been degraded in the oral thin film and/or that less than 2.5% of the contained adrenaline has been racemized.

13. Method for preparing an oral thin film according to any one of claims 1 to 12, comprising the steps of
a) preparing a solution, dispersion or melt comprising adrenaline or a pharmaceutically acceptable salt thereof and at least one polymer and
a1) optionally foaming the solution, dispersion or melt from step a) by introducing a gas or gas mixture, by chemical gas generation or by expansion of a dissolved gas,
b) spreading the solution, dispersion or melt from step a) or the optionally foamed solution, dispersion or melt from step a1) in order to obtain an oral thin film, and
(c) drying the oral thin film obtained in step (b) in order to obtain an oral thin film having a water activity of less than 0.6.

14. Oral thin film obtainable by the method according to claim 13.

15. Oral thin film according to any one of claims 1 to 12 or 14 for use as a medicament, particularly for use as a medicament in the treatment of allergic reactions.
